# EUROPEAN PATENT APPLICATION

(11) **EP 2 033 681 A1**
(43) Date of publication of application: **11.03.2009**
(21) Application number: 07115928.9
(22) Date of filing: 07.09.2007
(51) Int. Cl.: A61M 21/02

(54) **Bedtime product**

(71) Applicant: Koninklijke Philips Electronics N.V., 5621 BA Eindhoven (NL)
(72) Inventor: The designation of the inventor has not yet been filed
(74) Representative: de Haan, Poul Erik

(57) **Abstract**

The present invention relates to a bedtime product for improving sleep quality of a person comprising a housing, one or more electronic stimulus devices, an electronic control unit, adapted to activate the one or more stimulus devices in response to a signal and one or more sensors adapted to produce the signal. The invention is characterized by a clock providing an actual time signal and a memory, adapted to store a plurality of stimulus device instructions associated with an actual time or time interval, which stimulus device instructions include information on which one or more electronic stimulus devices is to be activated in response to a signal produce by the one or more sensors. The control unit is configured to select one of the stimulus device instructions based on the actual time signal and activate the one or more stimulus devices accordingly.

## Description

### FIELD OF THE INVENTION

The present invention relates to a bedtime product for improving sleep quality of a person, in particular a child, comprising
- a housing;
- one or more electronic stimulus devices, arranged in the housing, adapted to produce a stimulus stimulating at least one of the sensory organs of the person;
- an electronic control unit, arranged in the housing, adapted to activate the one or more stimulus devices in response to a signal;
- one or more sensors connected to the electronic control unit, adapted to produce the signal.

### BACKGROUND OF THE INVENTION

Such bedtime products are well known from prior art. An exemplary product is a teddy bear in which batteries, a speaker, an electronic control unit and a touch sensor are provided. When the touch sensor is activated, a signal is sent to the control unit which activates the speaker to play, for example, a night song. Sound is a stimulus which may comfort a child in the dark and thereby improve the sleep quality. Alternatively or in addition, such a speaker may be activated upon noise, e.g. a crying child, registered by a sound sensor.

An alternative exemplary bedtime product is a nightlight, comprising a clock and a time switch activating the nightlight. Alternatively, the nightlight may be activated by a light sensor, which activates the nightlight when it senses a lack of light.

### SUMMARY OF THE INVENTION

The invention is based on the insight that the right behavior of the bedtime product can be effective to improve a persons sleep quality. The aim of the present invention is to further improve the sleep quality of a person, in particular a child.

This aim is achieved by providing a bedtime product according to the preamble of claim 1, characterized by a clock connected to the electronic control unit and providing an actual time signal, and a memory, connected to the control unit, adapted to store a plurality of stimulus device instructions associated with an actual time or time interval, which stimulus device instructions include information on which one or more electronic stimulus devices is to be activated in response to a signal produce by the one or more sensors, wherein the control unit is configured to select one of the stimulus device instructions based on the actual time signal and activate the one or more stimulus devices accordingly.

The advantage of a bedtime product according to the invention is that the stimulus device instructions may differ for specific moments of the night. The behavior of the bedtime product is determined by a set of stimulus device instructions resulting in a stimulus stimulating at least one of the sensory organs. According to the invention, different behavior may be shown depending on the time of the day/ night. Appropriate behavior of the bedtime product resulting in the right stimulus, occurring at the right time in the evening, morning or during the night can be effective to improve the sleep quality of a person, in particular a child. This enables the bedtime product to interact in different phases of a sleep process in different ways, which fit the context of use.

Another advantage of the bedtime product according to the invention is that the stimulus may be produced by multiple combined stimulus devices, such as moving parts, sound and light. Conventional bedtime products often contain only one or two functions.

In the memory a plurality of stimulus device instructions is stored associated with an actual time or time interval. A combination of stimulus device instructions known to be effective for specific moments may be called a stimulus theme. The stimulus device instructions e.g. comprise information about the specific combination of one or more stimulus devices, the intensity of activation of the one or more stimulus devices, the duration of activation of the one or more stimulus devices and the rhythm of activation of the one or more stimulus devices. Such instructions may contain information on the color of light and its intensity, and the type of sound, e.g. a voice or a song or a breathing sound. In a preferred embodiment of the invention, the sound may be a recorded voice or song. The bedtime product may comprise a stimulus device such as a speaker which is activated by a touch sensor, and also a stimulus device such as a light which is set to be 'standard on' during a time interval. Alternatively, the stimulus device instructions may activate a sound stimulus device for a set time interval, and enable a motion stimulus device to be activated by a touch sensor, while a light stimulus device is activated by a light sensor to give a light during the entire time interval. At another time interval, the stimulus device instructions may both permanently activate the sound stimulus device and the motion stimulus device, while the light stimulus device is activated by the touch sensor. Other time intervals may be provided with yet alternative stimulus device instructions. The vast amount of stimulus device instructions that is possible to store in the memory enable a broad use of the bedtime product and increase the effectiveness of the product for improving sleep quality.

For example, a bedtime product may have a touch sensor and a light and music stimulus device. The level of the light and music used before a child goes to sleep can be actually very disturbing when the child wakes up in the middle of the night and touches the sensor of the bedtime product. In the bedtime product according to the invention a stimulus theme may be activated during the night with a lowered intensity of the stimulus devices.

Persons, in particular children, having sleep problems are sensitive to different types of stimulus at different times of a sleep process. This may vary from child to child, and may also change when children grow older. In a preferred embodiment, the memory is programmable to adapt the stimulus device instructions in the memory to a user's preferences. Such a customizable product enables parents to pre-select functions for their children, according to their life styles and personal preferences. The user may change the time setting, e.g. change the interval in which a set of stimulus device instructions is set. Also the stimulus device instructions may be adapted, e.g. by fine-tuning one or more stimulus device instructions such as decreasing the intensity of one or all stimulus devices, or switching on or off one or more stimulus devices. For example, the music level and light intensity may be lowered with respect to the pre-set stimulus device instructions, or the movements may be switched off. Preferably, it is possible both to adapt a specific set of stimulus device instructions associated with an actual time or time interval, but also to adapt overall settings, such as lowering all volume levels or increasing all light levels.

In a preferred embodiment, the bedtime product comprises multiple characters, each character being associated with a full set of stimulus device instructions for every actual time, during 24 hours. Such a character may for example be introvert, extravert, dreamy, active, girlish, boyish, etc. All stimulus themes (combinations of stimulus device instructions) present in the memory associated with such a character may be programmed to match the character: in colors, in dynamics, in type of songs, etc. etc.

In a preferred embodiment, the memory comprises stimulus device instructions mimicking various periods or phases of a sleep process. The instructions are e.g. associated with time intervals matching 'day', 'going to bed', 'fall asleep', 'wake up in night: go back to sleep', 'wake up in morning'. Possibly the stimulus device instructions comprise information on adapted intensity of activation of the one or more stimulus devices, such as dim lights, music volume etc. Alternatively, or in addition, the dynamics of activation of the one or more stimulus devices is adjusted, such as speed of color changing, music beat.

A possible memory setting may be as follows:
- Day (no need to sleep): high intensity of stimulus device, no fading.
- Going to bed (undress, story, etc): gradually fading stimulus device from high to low intensity when no longer activated.
- Need to fall asleep: low intensity of stimulus device and gradually fading stimulus device when no longer activated.
- Wake up in night, go back to sleep: quick fading on of the stimulus device when activated, to a low intensity of the stimulus device, and gradually fading out the stimulus device when no longer activated.
- Need to wake up in morning: gradually increasing the intensity of the stimulus device to a high intensity, and increasing the dynamics of the stimulus device.

Preferably, the stimulus device instructions comprise a setting in which the one or more stimulus devices produce a stimulus mimicking sleep and wake behavior. This is advantageous for children: seeing their teddy sleep (lights off, sleeping attitude, making a sound of a breathing person, closing eyes) will comfort them and stimulate children to sleep also. Preferably, the stimulus device instructions comprise a setting in which the one or more stimulus devices produce a stimulus with a rhythm that mimics the breathing rhythm of an average person falling asleep: not only a breathing sound but also faint lights fading and lighting up at the same rhythm.

Exemplary stimuli produced by stimulus devices according to the invention are light, music, movement, visuals (picture, animation, video), smell, temperature, humidity, etc. A light stimulus may be produced by a single light bulb, by multiple lights with different colors or alternatively a light patterns may be produced by photonic textile. Such an electronic device is arranged in the housing, e.g. inside a plastic object, or, in the case of photonic textile, integrated with the housing, e.g. the belly of a teddy bear. A sound stimulus may be music, a voice or e.g. a breathing sound produced by a speaker coupled to a sound memory. Movements may be created by moving parts of the housing, which are operated e.g. by servo motors arranged in the housing. Such a servo motor may be provided in the head of a teddy bear enabling the teddy bear to move its head or eyes upon activation of the stimulus device.

For instance, when a child is going to sleep, the stimulus device instructions of the bedtime product activate the stimulus devices to sing music, move repetitively and change light. And when a child is waking up during the night, the stimulus device instructions are similar but with a lowered stimulus energy, so that the light and music are at a very low level when the child is sleeping. In the morning, at the time when a child should wake up, the stimulus device instructions have and increased level of stimulus played, so that it helps a child wake up at the right time.

Exemplary sensors activating the one or more stimulus devices (via the electronic control unit) include touch sensors, e.g. buttons or sensors embedded in the housing, but also light sensors, motion sensors, remotely operable sensors or biometric sensors, such as hart beat sensors provided around the wrist of a person, or sensors measuring skin humidity or blood pressure. This enables activation of a stimulus device, such as a light, by a signal produced by a child pressing a button, but also by a signal produced by the parents pressing a button on the remote control. Biometric sensors activating the stimulus devices put the person in a more passive position. This enables the production of light patterns displayed at a rhythm synchronous with the breathing rhythm of the person.

Exemplary housings according to the invention include teddy bears and other fur animals or plastic creatures. Such housings may be cordless and comprise batteries for the operation of the electronic stimulus device, to enable the housings to be accommodated in the bed of the person. Alternatively, the housing may be positioned close to the bed and connected to the power network.

### BRIEF DESCRIPTIONOF THE DRAWINGS

The invention is further explained with reference to the drawings, in which:
fig. 1 represents a bedtime product with five activated stimulus devices;
fig. 2 gives a schematic representation of the invention;
figs. 3a-3d show the bedtime product of fig. 1 at three different time intervals with one or more stimulus devices activated based on stimulus device instructions in the memory.

### DETAILED DESCRIPTION OF THE EMBODIMENTS

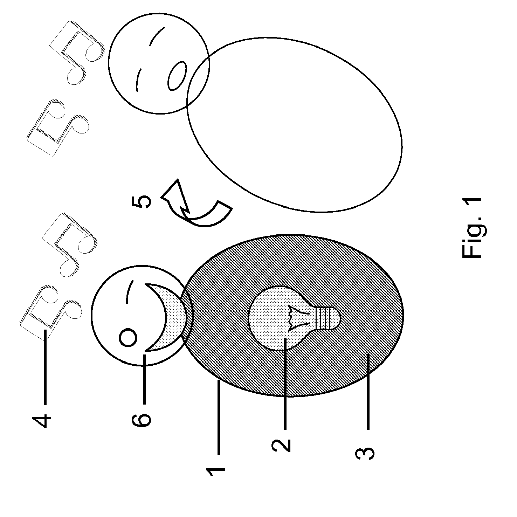

In fig. 1 a bedtime product according to the invention is schematically shown. The product comprises a housing (1) which is in the shown embodiment is a schematically depicted doll, but can be any type of toy or device. In the housing (1) a light bulb (2) is present which may be activated or not, or with different intensities. The housing (1) itself may in addition be lighted by LED lights (3) with different colors. Furthermore, the bedtime product may produce different sounds (4), move (5), and have a facial expression (e.g. open / close eyes, smile).

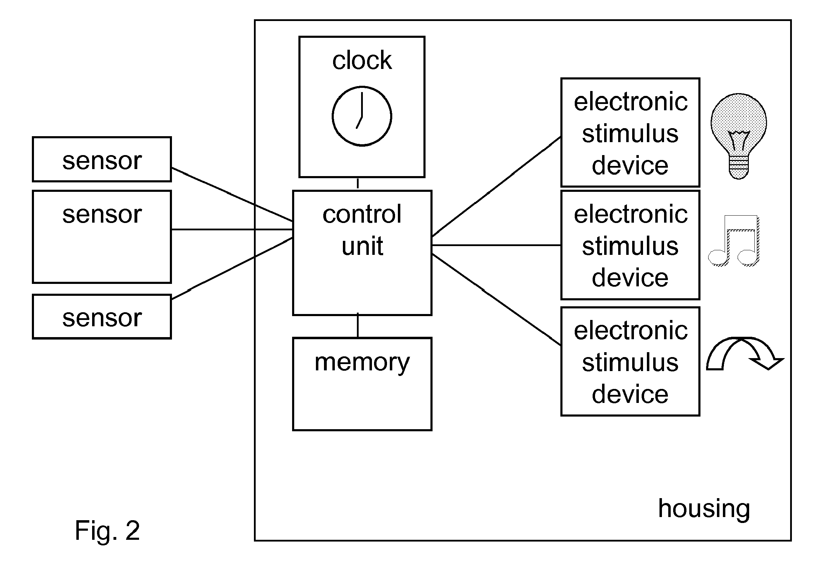

In fig. 2 the bedtime product for improving sleep quality of a person, in particular a child, according to the invention is schematically shown. The product comprises a housing in which one or more electronic stimulus devices are arranged, which are adapted to produce a stimulus. Examples of stimuli stimulating at least one of the sensory organs of the person are light, sound, and movement, which may be produced by electronic stimulating devices such as light sources, speakers and motors respectively. Also arranged in the housing is an electronic control unit which activates the one or more electronic device stimuli in response to a signal. Such a signal is produced by a sensor, which is connected to the electronic control unit. The sensor may be provided inside the housing, but also be provided outside the housing and connected to the control unit via a wireless connection. A clock is connected to the control unit, providing the control unit with the actual time. The control unit selects a set of stimulus device instructions based on the actual time and activates the one or more stimulus devices accordingly. The set of stimulus device instructions includes information about which stimulus devices are to activated, preferably also for which duration and with which intensity, in response to a signal produce by the one or more sensors.

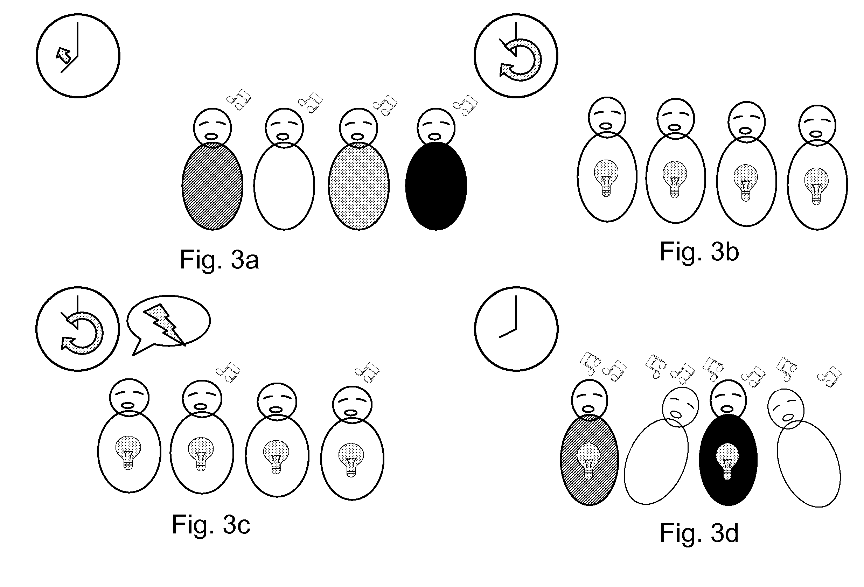

An example of the operation of a bedtime product according to the invention is shown in fig. 3.

At eight o'clock, the stimulus device instructions activate the speaker to produce a first song. The instructions may be such that the song is continuously played, with a decreasing sound level, for e.g. one hour. In addition, the stimulus device instructions may be set thus that the housing of the bedtime product is lighted with different colors when a signal produced by a light sensor is received. Such a light sensor may produce a signal when there is a lack of surrounding lights. Hence, when there is little surrounding lights, the teddy's belly will change colors. In case of sufficient surrounding lights, the housing will not lighten up.

During the night, from ten o'clock at night until seven o'clock in the morning, a faint light night is permanently produced by a light bulb in the bedtime product. Upon activation of a sound sensor, the stimulus device instructions may activate the bedtime product to play the same first song at a lower volume level, and also give a slight change of light a well: e.g. a very slowly change between two dimmed colors. The sound sensor may be activated when a significant noise is sensed, e.g. of a crying child or alternatively produced by lightning.

At seven thirty in the morning (7:30 AM), the stimulus device instructions may activate the light of the product to slowly increase to a higher intensity. This continues for a set time interval. This setting generates a gentle 'wake up phase'. At eight o'clock in the morning, the stimulus device instructions activate the movement stimulus device of the bedtime product, together with the sound generating device yet generating an alternative wake-up sound. The light bulb in the bedtime product is activated to start flashing, while the housing of the bedtime product is lighted with different colors. The stimulus devices flashing the light bulb and moving the product may be activated for a set time interval, e.g. for ten minutes from eight o'clock until 08.10. These devices may be re-activated for another 10 minutes in response to a signal produced by a sensor, such as a touch sensor on the housing which may be activated by the child, or by a remote control operated by the parents.

## Claims

1. Bedtime product for improving sleep quality of a person, in particular a child, comprising
- a housing (1);
- one or more electronic stimulus devices , arranged in the housing, adapted to produce a stimulus stimulating at least one of the sensory organs of the person;
- an electronic control unit, arranged in the housing, adapted to activate the one or more stimulus devices in response to a signal;
- one or more sensors connected to the electronic control unit, adapted to produce the signal;
**characterized by**
- a clock connected to the electronic control unit and providing an actual time signal,
- a memory, connected to the control unit, adapted to store a plurality of stimulus device instructions associated with an actual time or time interval, which stimulus device instructions include information on which one or more electronic stimulus devices is to be activated in response to a signal produced by the one or more sensors,
- wherein the control unit is configured to select one of the stimulus device instructions based on the actual time signal and activate the one or more stimulus devices accordingly.

2. Bedtime product according to claim 1, wherein the memory is programmable to adapt the stimulus device instructions in the memory to a user's preferences.

3. Bedtime product according to claim 1, in which the stimulus device instructions contain information about the combination of one or more stimulus devices, the intensity of activation of the one or more stimulus devices, the duration of activation of the one or more stimulus devices, and the rhythm of activation of the one or more stimulus devices.

4. Bedtime product according to claim 1, in which the sound stimulus produced by a speaker is customizable by recording a sound.

5. Bedtime product according to claim 1, in which the memory comprises stimulus device instructions associated with time intervals matching 'day', 'going to bed', 'fall asleep', 'wake up in night: go back to sleep' and 'wake up in morning'.

6. Bedtime product according to claim 1, in which the one or more sensors comprise a light sensor, a motion sensor, a remotely operable sensor, a touch sensor or a biometric sensor-control interface.

7. Bedtime product according to claim 1, in which the one or more stimulus devices comprise a light, a sound generator, a fragrance generator, a temperature generator, a humidity generator, one or more moveable components or a sound generator playing a breathing sound

8. Bedtime product according to claim 1, the stimulus device instructions comprise a setting in which the one or more stimulus devices produce a stimulus mimicking sleep and wake behavior.

## Amended claims

### Amended claims in accordance with Rule 137(2) EPC.

**1.** Bedtime product for improving sleep quality of a person, in particular a child, comprising
- a housing;
- one or more electronic stimulus devices , arranged in the housing, adapted to produce a stimulus stimulating at least one of the sensory organs of the person;
- an electronic control unit, arranged in the housing, adapted to activate the one or more stimulus devices in response to a signal;
- one or more sensors connected to the electronic control unit, adapted to produce the signal;
**characterized by**
- a clock connected to the electronic control unit and providing an actual time signal,
- a memory, connected to the control unit, adapted to store a plurality of stimulus device instructions associated with an actual time or time interval, which stimulus device instructions include information on which one or more electronic stimulus devices is to be activated in response to a signal produced by the one or more sensors,
- wherein the control unit is configured to select one of the stimulus device instructions based on the actual time signal and activate the one or more stimulus devices accordingly,
the stimulus device instructions comprising a setting in which the one or more stimulus devices produce a stimulus with a rhythm that mimics the breathing rhythm of an average person falling asleep.

**2.** Bedtime product according to claim 1, wherein producing a stimulus with a rhythm that mimics the breathing rhythm of an average person falling asleep comprises making a sound of a breathing person at the same rhythm.

**3.** Bedtime product according to claim 1 or 2, wherein producing a stimulus with a rhythm that mimics the breathing rhythm of an average person falling asleep comprises lights fading and lighting up at the same rhythm.

**4.** Bedtime product according to claim 1, wherein the memory is programmable to adapt the stimulus device instructions in the memory to a user's preferences.

**5.** Bedtime product according to claim 1, in which the stimulus device instructions contain information about the combination of one or more stimulus devices, the intensity of activation of the one or more stimulus devices, the duration of activation of the one or more stimulus devices, and the rhythm of activation of the one or more stimulus devices.

**6.** Bedtime product according to claim 1, in which the sound stimulus produced by a speaker is customizable by recording a sound.

**7.** Bedtime product according to claim 1, in which the memory comprises stimulus device instructions associated with time intervals matching 'day', 'going to bed', 'fall asleep', 'wake up in night: go back to sleep' and 'wake up in morning'.

**8.** Bedtime product according to claim 1, in which the one or more sensors comprise a light sensor, a motion sensor, a remotely operable sensor, a touch sensor or a biometric sensor-control interface.

**9.** Bedtime product according to claim 1, in which the one or more stimulus devices comprise a light, a sound generator, a fragrance generator, a temperature generator, a humidity generator, one or more moveable components or a sound generator playing a breathing sound.
